# EUROPÄISCHE PATENTSCHRIFT

(11) **EP 3 636 219 B1**
(45) Veröffentlichungstag und Bekanntmachung des Hinweises auf die Patenterteilung: **18.01.2023**
(21) Anmeldenummer: 19194640.9
(22) Anmeldetag: 30.08.2019
(51) Int. Cl.: A61C 19/10, A61C 13/00, A61C 13/08

(54) **VERFAHREN ZUM HERSTELLEN EINES ZAHNERSATZTEILS**
METHOD FOR PRODUCING A DENTAL PROSTHETIC
PROCÉDÉ DE FABRICATION D'UNE PROTHÈSE DENTAIRE

(30) Priorität: 10.10.2018 EP 18199590
(43) Veröffentlichungstag der Anmeldung: 15.04.2020
(73) Patentinhaber: Ivoclar Vivadent AG, 9494 Schaan (LI)
(72) Erfinder: Ritzberger, Christian, 9472 Grabs (CH)
(74) Vertreter: Baldus, Oliver

(56) Entgegenhaltungen:
- US-A- 4 654 794
- US-A1- 2012 052 186
- US-A1- 2012 261 848
- US-A1- 2015 086 939
- US-A1- 2015 182 317
- US-A1- 2016 302 896

## Beschreibung

Die vorliegende Erfindung betrifft ein Verfahren zum Herstellen eines Zahnersatzteils und ein Computerprogramm.

Zahnersatzteile lassen sich auf Computern mit entsprechenden Benutzerschnittstellen (CAD/CAM) farblich und räumlich gestalten. Bei diesem Gestaltungsprozess wird versucht, Farbwerte zu ermitteln, die den Zahnersatz möglichst natürlich und abgestimmt auf die verbleibenden Zähne des Patienten erscheinen zu lassen. Diese Farbwerte können mittels einer farblichen Erfassung, d.h. Farbabnahme, der Nachbarzähne und/oder des verbleibenden durch den Zahnarzt präparierten vitalen oder de-vitalen Zahnstumpfes ermittelt werden.

Allerdings unterscheiden sich die Farben einer Färbeflüssigkeit zum Einfärben einer teilgesinterten porösen, dentalen Restauration für den späteren Zahnersatz eklatant von den zuvor ausgewählten natürlichen Farben. Erst im Laufe des Herstellungsprozesses, wie beispielsweise beim Sintern, verliert die Färbeflüssigkeit für das Zahnersatzteil ihre ursprüngliche Farbe und nimmt diejenige natürliche Farbe an, die für den Zahnersatz ausgewählt oder berechnet worden ist. Die Färbeflüssigkeiten sind Lösungen mit Ionen der 3d und/oder 4f-Elemente, die beim Sintern ins ZrO₂-Gitter eingebaut werden und die Färbung bewirken. Die Färbeflüssigkeit umfasst eine oder mehrere Lebensmittelfarben zur Unterscheidung , die beim Sintern ausgebrannt wird.

Es ist daher die technische Aufgabe der vorliegenden Erfindung, die Herstellung eines Zahnersatzteils technisch zu vereinfachen und zu beschleunigen.

Das Dokument US4654794 offenbart ein Verfahren zur Bestimmung der richtigen Färbung für eine Zahnnachbildung.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird diese Aufgabe durch ein Verfahren zum Herstellen eines Zahnersatzteils gelöst, mit den Schritten eines Ermittelns eines natürlichen Zahnfarbwertes zu einem vorgegebenen Bereich des Zahnersatzteils; und eines elektronischen Umrechnens des natürlichen Zahnfarbwertes in einen Farbwert einer Färbeflüssigkeit zum Färben des Bereiches.

Das Verfahren kann auch auf mehrere Bereiche des Zahnersatzteils mit unterschiedlichen Färbelösungen angewendet werden. Das Zahnersatzteil ist beispielsweise eine endkonturnahe, poröse dentale Restauration, die vorzugsweise aus yttriumstabilisiertem Zirkonoxid gebildet ist. Die endkonturnahe, poröse dentale Restauration ist 15-20% größer als nach dem Dichtsintern, weist aber bereits die vorgesehene Form einer Krone, einer Brücke, eines Abutments oder eines Implantates auf. Durch das automatische Umrechnen der zugewiesenen natürlichen Zahnfarbwerte in Farbwerte einer Färbeflüssigkeit für die endkonturnahe, poröse dentale Restauration kann die Herstellung des Zahnersatzteils vereinfacht und individuell eingefärbt werden. Bei einer 7D-Farbgebung wird die Farbgebung in 7 Dimensionen berücksichtigt, drei Dimensionen (3D) für alle Raumrichtungen, eine Dimension (1D) für den a*-Wert, eine Dimension (1D) für den b*-Wert, eine Dimension (1D) für CR-Wert und eine Dimension (1D) für L*-Wert.

In einer technisch vorteilhaften Ausführungsform des Verfahrens zum Herstellen des Zahnersatzteils wird der vorgegebene Bereich des Zahnersatzteils automatisch mit derjenigen Färbeflüssigkeit eingefärbt, die den umgerechneten Farbwert aufweist. Dadurch wird beispielsweise der technische Vorteil erreicht, dass sich die Herstellung des Zahnersatzteils noch weiter beschleunigt. In dieser technisch vorteilhaften Ausführungsform des Verfahrens zum Herstellen des Zahnersatzteils kann dies direkt in der CAD/CAM Maschine nach dem Fräsen der endkonturnahen, porösen dentalen Restauration erfolgen, indem die CAD/CAM-Vorrichtung mit einer Farbauftragsvorrichtung kombiniert wird.

In einer weiteren technisch vorteilhaften Ausführungsform des Verfahrens zum Herstellen des Zahnersatzteils wird das eingefärbte Zahnersatzteil optisch abgetastet, um den Ist-Farbwert der Einfärbung des Zahnersatzteils mit der Färbeflüssigkeit zu ermitteln. Dadurch wird beispielsweise der technische Vorteil erreicht, dass sich die korrekte Einfärbung des Zahnersatzteils überprüfen lässt.

In einer weiteren technisch vorteilhaften Ausführungsform des Verfahrens zum Herstellen des Zahnersatzteils wird der ermittelte Ist-Farbwert in den zugeordneten natürlichen Zahnfarbwert umgerechnet. Dadurch wird beispielsweise ebenfalls der technische Vorteil erreicht, dass sich das natürliche Aussehen des Zahnersatzteils auf Basis der tatsächlichen Einfärbung des Zahnersatzteils vorausberechnen lässt.

In einer weiteren technisch vorteilhaften Ausführungsform des Verfahrens zum Herstellen des Zahnersatzteils wird das Zahnersatzteil gesintert oder dichtgesintert. Dadurch wird beispielsweise der technische Vorteil erreicht, dass das einsetzbare Zahnersatzteil erhalten wird.

In einer weiteren technisch vorteilhaften Ausführungsform des Verfahrens zum Herstellen des Zahnersatzteils wird der Farbwert der Färbeflüssigkeit in einen Datensatz integriert, der die dreidimensionale Form des Zahnersatzteils beschreibt. Zusätzlich kann der Bereich der Einfärbung in den Datensatz integriert werden. Dadurch wird beispielsweise ebenfalls der technische Vorteil erreicht, dass sich die Herstellung des Zahnersatzteils vereinfacht, indem ein einziger Datensatz an ein Herstellungsgerät übermittelt wird.

In einer weiteren technisch vorteilhaften Ausführungsform des Verfahrens zum Herstellen des Zahnersatzteils wird der Datensatz auf Basis einer dreidimensionalen Modellierung des Zahnersatzteiles erhalten. Dadurch wird beispielsweise ebenfalls der technische Vorteil erreicht, dass sich der Datensatz auf einfache Weise bestimmen lässt.

In einer weiteren technisch vorteilhaften Ausführungsform des Verfahrens zum Herstellen des Zahnersatzteils wird der Datensatz an eine Druckstation zum Färben des Zahnersatzteils gesendet. Die Druckstation kann in einem CAD/CAM-Gerät integriert sein oder existiert als separates Gerät. Dadurch wird beispielsweise ebenfalls der technische Vorteil erreicht, dass sich die Herstellung des Zahnersatzteils weiter beschleunigt.

In einer weiteren technisch vorteilhaften Ausführungsform des Verfahrens zum Herstellen des Zahnersatzteils wird der Farbwert der Färbeflüssigkeit auf einer Datenbrille dem vorgegebenen Bereich des Zahnersatzteils visuell überlagert. Durch die Berechnung eines Farbschemas und einer anschließenden Darstellung über eine Augmented-Reality-App (AR-Application) oder Augmented-Reality-Datenbrille (AR-Datenbrille) kann einer Person die optimale dreidimensionale Farbanpassung zur Herstellung einer hochästhetischen dentalen Restauration angezeigt werden. Dadurch kann jeder Bereich (Punkt/Pixel/Fläche) individuell eingefärbt werden. Der Fokus ist die 100%ige individuelle Farbgebung und Farbanpassung an die Nachbarzähne unter zu Hilfenahme der AR-Anwendung (Malen nach Zahlen) unter vorheriger Einbeziehung der Farbe der Nachbarzähne und des präparierten Stumpfes. Das ästhetische Resultat wird unter Zuhilfenahme der beschriebenen AR-Merkmale erreicht.

Dadurch wird beispielsweise der technische Vorteil erreicht, dass auf Basis des überlagerten Falschfarbenbildes das Zahnersatzteil mittels einer Pinselinfiltration manuell eingefärbt werden kann.

In einer weiteren technisch vorteilhaften Ausführungsform des Verfahrens zum Herstellen des Zahnersatzteils wird der Farbwert der Färbeflüssigkeit dem vorgegebenen Bereich des Zahnersatzteils auf einem tragbaren Anzeigegerät visuell überlagert wird. Dadurch wird beispielsweise ebenfalls der technische Vorteil erreicht, dass auf Basis des überlagerten Falschfarbenbildes das Zahnersatzteil mittels einer Pinselinfiltration manuell eingefärbt werden kann.

In einer weiteren technisch vorteilhaften Ausführungsform des Verfahrens zum Herstellen des Zahnersatzteils wird der natürliche Zahnfarbwert durch eine farbliche Abtastung eines vorhandenen Zahns und/oder Zahnstumpfes erhalten. Dadurch wird beispielsweise ebenfalls der technische Vorteil erreicht, dass sich die natürlichen Zahnfarbwerte auf einfache Weise ermitteln lassen.

In einer weiteren technisch vorteilhaften Ausführungsform des Verfahrens zum Herstellen des Zahnersatzteils wird dem vorgegebenen Bereich des angezeigten Zahnersatzteils eine Farbtiefe zugeordnet und die Farbtiefe in eine Anzahl an Auftragungen der Färbeflüssigkeit umgerechnet. Dadurch wird beispielsweise ebenfalls der technische Vorteil erreicht, dass das die gewünschte Farbtiefe des Zahnersatzteils auf einfache Weise ermittelt werden kann.

Gemäß einem zweiten Aspekt wird die Aufgabe durch eine Vorrichtung gelöst, umfassend Mittel zur Ausführung des Verfahrens nach dem ersten Aspekt. Dadurch lassen sich die gleichen technischen Vorteile wie durch das Verfahren nach dem ersten Aspekt erreichen.

Gemäß einem dritten Aspekt wird die Aufgabe durch ein Computerprogramm gelöst, umfassend Befehle, die bei der Ausführung des Computerprogramms durch einen Computer diesen veranlassen, das Verfahren nach dem ersten Aspekt auszuführen. Dadurch lassen sich ebenfalls die gleichen technischen Vorteile wie durch das Verfahren nach dem ersten Aspekt erreichen.

Ausführungsbeispiele der Erfindung sind in den Zeichnungen dargestellt und werden im Folgenden näher beschrieben.

Es zeigen:
- Fig. 1: eine schematische Ansicht eines Zahnersatzteils; und
- Fig. 2: ein Blockdiagramm des Verfahrens.

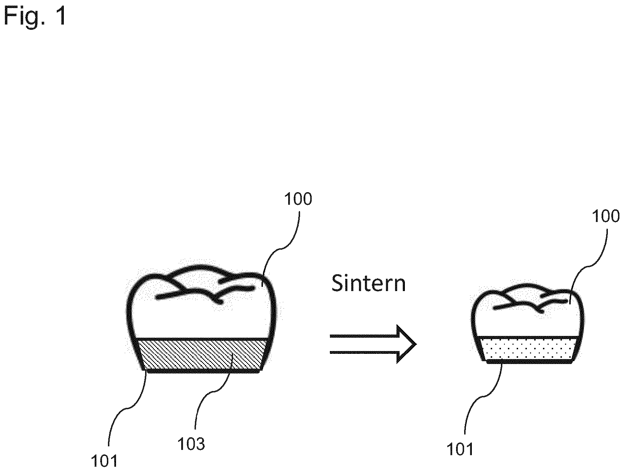

Fig. 1 zeigt eine schematische Ansicht eines Zahnersatzteils 100. Das Zahnersatzteil 100 ist beispielsweise eine Krone, eine Brücke, ein Abutment oder ein Implantat, das im Rahmen einer Zahnbehandlung einem Patienten als Zahnersatz eingesetzt wird. Im Allgemeinen soll die Behandlungszeit bei der Zahnbehandlung so gering wie möglich gehalten werden. Daher sollte das Zahnersatzteil 100 in möglichst kurzer Zeit zur Verfügung stehen.

Das Herstellen des Zahnersatzteils 100 erfolgt unter anderem dadurch, dass das gefräste Zahnersatzteil 100 in einem Sinterofen gesintert wird. Die endkonturnahe, dentale Restauration ist 15-20% grösser als nach dem Dichtsintern, weist aber bereits die später vorgesehene Form auf. Beim Dichtsintern nimmt die endkonturnahe, dentale Restauration die vorgesehene Größe, Dichte, Festigkeit und Zähigkeit an.

Vorgegebene Bereiche 101 des Zahnersatzteils 100 sind mit einer Färbeflüssigkeit 103 eingefärbt, die erst im Laufe des Sinterprozesses in die vorgesehene natürliche Zahnfarbe übergeht. Im Gegensatz zu den späteren natürlichen Zahnfarben, die sich oftmals nur in Nuancen unterscheiden, haben die unterschiedlichen Färbeflüssigkeiten 103 für das Zahnersatzteil 100 optisch gut unterscheidbare Farben, wie beispielsweise Chinolingelb und Patentblau oder der Farbe entsprechen, die durch den Einsatz von notwendigen Salzen der 3d- und 4f-Element in der Färbelösung ergeben. Die Farbwirkung im gesinterten Zahnersatzteil 100 wird durch den Ioneneinbau der Elemente wie beispielsweise Fe, Er, Tb, Tm, Pr, Co, Cr, Ni, Mn oder Bi im Kristallgitter des tetragonalen und/oder kubischem Zirkonoxids erzeugt. Das teil- oder vollstabilisierte tetragonale und/oder kubische Zirkonoxid ist üblicherweise mit Y203, La203, Ce02, MgO oder CaO oder deren Mischungen stabilisiert. In den Färbeflüssigkeiten 103 sind die Salze, wie z. B. Chloride, Nitrate, der 3d- und 4f-Elemente gelöst. Die farbliche Unterscheidung der Färbeflüssigkeiten erfolgt durch die Verwendung geeigneter Lebensmittelfarben.

Das Bestimmen und Auftragen der zu verwendenden Färbelösungen 103 bei der Herstellung des Zahnersatzteils ist zeitaufwändig. Allerdings sollte die Herstellungszeit für das Zahnersatzteil 100 so verkürzt werden, dass eine Zahnbehandlung mit dem Zahnersatz beispielsweise an einem halben Tag durchgeführt werden kann.

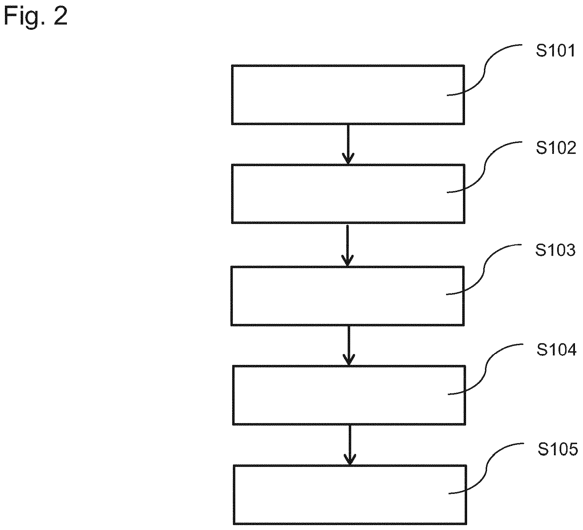

Fig. 2 zeigt ein Blockdiagramm des Verfahrens zum beschleunigten Herstellen des Zahnersatzteils 100.

In Schritt S101 wird zunächst ein spezieller Rohling aus Zirkonoxid in Abhängigkeit der jeweiligen zahnmedizinischen Indikation, wie beispielsweise bei einem Einsatz als Front- oder Seitenzahn, Krone oder Brücke, mit Halter zum Fräsen bereitgestellt. Dieser Rohling wird auch als Weißling bezeichnet, da dieser nicht voreingefärbt ist. Der Rohling ist entbindert und bereits vorgesintert.

Für die verschiedenen zahnmedizinischen Indikationen werden lediglich drei verschiedene Zirkonoxid-Rohlinge verwendet, die sich insbesondere in deren Y₂O₃-Anteil unterscheiden. Der Y₂O₃-Anteil hat einen Einfluss auf die Transluzenz und die Festigkeit des endgültigen Zahnersatzteils 100. Neben Y₂O₃ können auch andere Oxide zur Stabilisierung eingesetzt werden, wie z.B. Oxide des Ce, Mg, Ca und La sowie Mischungen daraus verwendet werden. Ein erster Typ des Zirkonoxid-Rohlings umfasst 2.5 - 3.5 Mol-% yttriumstabilisiertes Zirkonoxid. Ein zweiter Typ des Zirkonoxid-Rohlings umfasst 3.5 - 4.5 Mol-% yttriumstabilisiertes Zirkonoxid. Ein dritter Typ des Zirkonoxid-Rohlings umfasst 4.5 - 5.5 Mol-% yttriumstabilisiertes Zirkonoxid. Je nach zahnmedizinischer Indikation wird aus den drei unterschiedlichen Zirkonoxid-Rohlingen ausgewählt. Diese Auswahl kann durch einen Zahnarzt oder eine geeignete Software schnell und einfach getroffen werden.

In Schritt S102 wird die Form des Zahnersatzteils 100 festgelegt. Hierzu wird der zu versorgende Zahnbereich mit einem oder mehreren Zähnen präpariert. Nach Abschluss der Präparation wird ein digitaler Scan mittels einer Interoralkamera durchgeführt. Mit den digitalen Daten wird die Restauration, d.h. das Zahnersatzteil 100, mittels eines CAD-Programms modelliert. Anschließend wird aus dem zuvor ausgewählten Zirkonoxid-Rohling in einem CAD/CAM Prozess die modellierte Form des dentalen Zahnersatzteils 100 gefräst.

Das so gewonnene Zahnersatzteil 100 ist durch eine endkonturnahe, teilgesinterte poröse dentale Restauration gebildet. Die endkonturnahe, dentale Restauration ist 15-20% größer als nach dem späteren Dichtsintern und weist bereits die vorgesehene Form der späteren Krone, der Brücke, des Abutments oder des Implantates auf.

Im Schritt S103 werden die natürlichen Zahnfarbwerte für vorgegebene Bereiche 101 des Zahnersatzteils 100 ermittelt. Das Ermitteln der natürlichen Zahnfarbwerte für das Zahnersatzteil 100 kann durch eine Farbabnahme der Nachbarzähne und eines verbleibenden Zahnstumpfes auf Basis eines optischen Messverfahrens mit einer Kamera durchgeführt werden. Der Zahnstumpf kann vital und/oder de-vital sein. Anhand der dadurch gewonnenen Daten errechnet eine Software ein 3D-Modell für die individuelle natürliche Farbgebung und Form der dentalen Restauration, so dass sich diese farblich nahtlos in die verbliebenen Zähne einfügt.

Im Schritt S104 werden die ermittelten natürlichen Zahnfarbwerte elektronisch in Farbwerte von Färbeflüssigkeiten 103 zum Färben des Zahnersatzteils 100 vor dem Sintern umgerechnet. Das Umrechnen kann auf Basis einer vorgespeicherten Tabelle erfolgen, in der den jeweiligen natürlichen Zahnfarbwerten die jeweiligen Farbwerte der Färbeflüssigkeiten 103 zugeordnet sind. Die Farbwerte der Färbeflüssigkeiten 103 können in Form eines Bildes zur Verfügung gestellt werden, durch das der Zahnarzt eine einfache Anleitung bekommt, welche Bereiche 101 des Zahnersatzteils 100 mittels der Färbeflüssigkeiten 103, d.h. Infiltrationsfarben, wie einzufärben sind. Hierzu kann ein frei drehbares 3D-Modell an einem Bildschirm dargestellt werden, das die Farben der Färbeflüssigkeiten 103 für die jeweiligen Bereiche 101 auf einem Bildschirm anzeigt. Allerdings kann das 3D-Modell mittels einer AR-Software (AR - Augmented Reality) auf einem tragbaren Gerät mit Kamera, wie beispielsweise einem Smartphone oder Tablet, oder auf einer Datenbrille dargestellt werden und visuell dem Zahnersatzteil 100 überlagert werden. Dadurch kann ebenfalls auf einfache Weise erkannt werden, welche Bereiche 101 des Zahnersatzteils 100 mittels der Färbeflüssigkeiten 103 wie einzufärben sind.

Die Datenbrille ist ein als Brille getragenes elektronisches Gerät, mit dem einem Benutzer zusätzlich zur natürlichen visuellen Wahrnehmung des Zahnersatzteils 100 die Farbewerte für die jeweiligen Bereiche 101 optisch angezeigt werden können. Die Datenbrille kann durch die Anzeigeeinrichtung zusätzliche Informationen auf einem Bild überlagern, das von dem Auge des Trägers wahrgenommen wird. Die Anzeigeeinrichtung umfasst beispielsweise einen augennahen Bildschirm oder einen Projektor zur direkten Projektion auf der Netzhaut. In entsprechende Weise kann dies auch einem tragbaren Gerät erfolgen.

Das 3D-Modell ist so dargestellt, dass farbliche Bereiche 101 des angezeigten 3D-Modells der Farbe der Färbelösungen 103 entsprechen, mit denen das Zahnersatzteil 100 eingefärbt werden muss, um später das gewünschte natürliche Aussehen zu erhalten. Dadurch kann das Zahnersatzteil 100 auf einfache Weise entsprechend eingefärbt werden und die Zeit zur Herstellung des Zahnersatzteils 100 verringert werden.

Die vorgegebenen Bereiche 101 des Zahnersatzteils 100 können automatisch mit den jeweiligen Färbeflüssigkeiten 103 eingefärbt werden, die die umgerechneten Farbwerte aufweisen. Dies kann durch Verwendung eines Druckgerätes erfolgen, mit dem die Färbeflüssigkeiten 103 auf das Zahnersatzteil aufgebracht werden. Die Bereiche 101 können auch auf Basis des angezeigten Bildes manuell mittels Pinselinfiltration einfärbt werden. Alternativ können die Färbeflüssigkeiten 103 auch aufgesprüht werden, mit der Hilfe einer geeigneten Sprühvorrichtung.

Anschließend kann das eingefärbte Zahnersatzteil 100 optisch erneut mittels eines Messverfahrens abgetastet werden, um die Ist-Farbwerte der Einfärbung des Zahnersatzteils 100 mit den Färbeflüssigkeiten 103 zu ermitteln und mit den vorgesehenen Farbwerten der Färbeflüssigkeiten 103 zu vergleichen. Dabei kann überprüft werden, dass jeder der vorgegebenen Bereiche 101 mit der vorgesehenen Farbflüssigkeit eingefärbt ist. Dadurch kann eine korrekte Einfärbung überprüft werden. Die ermittelten Ist-Farbwerte der Einfärbung des Zahnersatzteils 100 mit den Färbeflüssigkeiten 103 können in die jeweils zugeordneten natürlichen Zahnfarbwerte zurück-umgerechnet werden. Auf diese Weise kann ein späteres Aussehen auf Basis des tatsächlich eingefärbten Zahnersatzteils 100 ermittelt und angezeigt werden. Das Messverfahren kann mit einer Kamera oder einem geeigneten RGB Sensor durchgeführt werden. Dadurch soll es möglich sein, die Farbgebung des Zahnersatzteils 100 festzustellen. Es können der a*-Wert, der b*Wert, der CR-Wert und der L*-Wert ermittelt werden. Denkbar ist es, dass nur ein Wert aus den vorhergenannten Werten ermittelt wird und in die jeweils zugeordneten natürlichen Zahnfarbwerte zurückgerechnet werden. Die Umrechnung kann von dem Computerprogramm durchgeführt werden und an einem Bildschirm angezeigt werden.

Die umgerechneten Farbwerte der Färbeflüssigkeit 103 und die zugehörigen Bereiche 101 können in einen Datensatz integriert werden, der die dreidimensionale Form des Zahnersatzteils 100 beschreibt. Dieser kann auf Basis einer dreidimensionalen Abtastung (Scan) einer Mundposition oder einer digitalen Modellierung erhalten werden. Dieser Datensatz kann anschließend an eine Druckstation oder ein Druckgerät zum automatischen Färben des Zahnersatzteils 100 gesendet werden.

Den vorgegebenen Bereichen 101 des Zahnersatzteils 100 kann automatisch oder mittels einer Benutzerschnittstelle jeweils eine Farbtiefe zugeordnet werden. Diese festgelegte Farbtiefe kann automatisch in eine Anzahl an Auftragungen der Färbeflüssigkeit 103 umgerechnet werden. Anschließend kann die Färbeflüssigkeit 103 entsprechend oft aufgetragen werden, beispielsweise mittels des Druckgerätes.

Im Schritt S105 wird das Zahnersatzteil 100 in einem Ofen getrocknet und gesintert. Die Trocknung kann in einem Ofen oder mit einer Infrarotquelle erfolgen. Da nur oberflächlich infiltriert wird, kann die Trocknung in einem zeitlichen Bereich von 10 s bis 10 min erfolgen. Durch das Sintern erhält das Zahnersatzteil 100 seine endgültige Form und die Färbelösungen 103 bewirken im dichtgesinterten Zustand die vorgesehenen natürlichen Zahnfarbwerte. Anschließend kann eine Oberflächenpolitur oder ein Glasurbrand zur Oberflächenveredelung erfolgen.

Durch das Verfahren wird es möglich, Patienten in kurzer Zeit mit einer dentalen Restauration aus Zirkonoxid zu versorgen. Diese weist eine Farbgebung auf, die der geplanten Farbgebung präzise entspricht.

Das Ermitteln und Umrechnen der natürlichen Zahnfarbwerte in Farbwerte von Färbeflüssigkeiten führt neben den anderen Maßnahmen zu einer erheblichen Verkürzung der Herstellung des Zahnersatzteils 100. Dadurch lässt sich eine Zahnbehandlung, bei der das Zahnersatzteil 100 verwendet wird, deutlich verkürzen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Verfahren zum Herstellen eines Zahnersatzteils (100), mit den Schritten:
Ermitteln (S103) eines natürlichen Zahnfarbwertes zu einem vorgegebenen Bereich (101) des Zahnersatzteils (100); und
elektronisches Umrechnen (S104) des natürlichen Zahnfarbwertes in einen Farbwert einer Färbeflüssigkeit (103) zum Färben des Bereiches (101), die eine oder mehrere Lebensmittelfarben zur Unterscheidung umfasst, die beim Sintern ausgebrannt werden.

2. Verfahren nach Anspruch 1, wobei der vorgegebene Bereich (101) des Zahnersatzteils (100) automatisch mit derjenigen Färbeflüssigkeit (103) eingefärbt wird, die den umgerechneten Farbwert aufweist.

3. Verfahren nach Anspruch 2, wobei das eingefärbte Zahnersatzteil (100) optisch abgetastet wird, um den Ist-Farbwert der Einfärbung des Zahnersatzteils (100) mit der Färbeflüssigkeit (103) zu ermitteln.

4. Verfahren nach Anspruch 3, wobei der ermittelte Ist-Farbwert in den zugeordneten natürlichen Zahnfarbwert umgerechnet wird.

5. Verfahren nach einem der vorangehenden Ansprüche, wobei das Zahnersatzteil (100) gesintert wird.

6. Verfahren nach einem der vorangehenden Ansprüche, wobei der Farbwert der Färbeflüssigkeit (103) in einen Datensatz integriert wird, der die dreidimensionale Form des Zahnersatzteils (100) beschreibt.

7. Verfahren nach Anspruch 6, wobei der Datensatz auf Basis einer dreidimensionalen Modellierung des Zahnersatzteiles (100) erhalten wird.

8. Verfahren nach Anspruch 7, wobei der Datensatz an eine Druckstation zum Färben des Zahnersatzteils (100) gesendet wird.

9. Verfahren nach einem der vorangehenden Ansprüche, wobei der Farbwert der Färbeflüssigkeit (103) auf einer Datenbrille dem vorgegebenen Bereich des Zahnersatzteils (100) visuell überlagert wird.

10. Verfahren nach einem der vorangehenden Ansprüche, wobei der Farbwert der Färbeflüssigkeit (103) dem vorgegebenen Bereich des Zahnersatzteils (100) auf einem tragbaren Anzeigegerät visuell überlagert wird.

11. Verfahren nach einem der vorangehenden Ansprüche, wobei der natürliche Zahnfarbwert durch eine farbliche Abtastung eines vorhandenen Zahns und/oder Zahnstumpfes erhalten wird.

12. Verfahren nach einem der vorangehenden Ansprüche, wobei dem vorgegebenen Bereich (101) des angezeigten Zahnersatzteils (100) eine Farbtiefe zugeordnet wird und die Farbtiefe in eine Anzahl an Auftragungen der Färbeflüssigkeit (103) umgerechnet wird.

## Claims

1. A method for producing a dental prosthesis part (100), comprising the steps of:
determining (S103) a natural tooth color value with respect to a specified region (101) of the dental prosthesis part (100); and
electronically converting (S104) the natural tooth color value into a color value of a coloring liquid (103) for coloring the region (101), said coloring liquid comprising one or more food colorings for distinction which are burnt out during sintering.

2. The method as claimed in claim 1, wherein the specified region (101) of the dental prosthesis part (100) is colored automatically with the particular coloring liquid (103) which has the converted color value.

3. The method as claimed in claim 2, wherein the colored dental prosthesis part (100) is optically scanned in order to determine the actual color value of the coloring of the dental prosthesis part (100) with the coloring liquid (103) .

4. The method as claimed in claim 3, wherein the determined actual color value is converted into the allocated natural tooth color value.

5. The method as claimed in any one of the preceding claims, wherein the dental prosthesis part (100) is sintered.

6. The method as claimed in any one of the preceding claims, wherein the color value of the coloring liquid (103) is integrated into a data record which describes the three-dimensional shape of the dental prosthesis part (100).

7. The method as claimed in claim 6, wherein the data record is obtained on the basis of a three-dimensional modelling of the dental prosthesis part (100).

8. The method as claimed in claim 7, wherein the data record is transmitted to a printing station for coloring the dental prosthesis part (100).

9. The method as claimed in any one of the preceding claims, wherein the color value of the coloring liquid (103) is visually superimposed on data glasses onto the specified region of the dental prosthesis part (100).

10. The method as claimed in any one of the preceding claims, wherein the color value of the coloring liquid (103) is visually superimposed on a wearable display apparatus onto the specified region of the dental prosthesis part (100).

11. The method as claimed in any one of the preceding claims, wherein the natural tooth color value is obtained by color-scanning an existing tooth and/or tooth stump.

12. The method as claimed in any one of the preceding claims, wherein a color depth is allocated to the specified region (101) of the displayed dental prosthesis part (100) and the color depth is converted into a number of applications of the coloring liquid (103).

## Revendications

1. Procédé de fabrication d'une prothèse dentaire (100), comprenant les étapes suivantes:
détermination (S103) d'une valeur de couleur de dent naturelle pour une zone prédéterminée (101) de la prothèse dentaire (100) ;
et conversion électronique (S104) de la valeur de couleur de dent naturelle en une valeur de couleur d'un liquide de coloration (103) pour colorer la zone (101), comprenant un ou plusieurs colorants alimentaires pour la différenciation, qui sont brûlés lors du frittage.

2. Procédé selon la revendication 1, dans lequel la zone prédéterminée (101) de la prothèse dentaire (100) est colorée automatiquement avec le liquide de coloration (103) qui présente la valeur de couleur convertie.

3. Procédé selon la revendication 2, dans lequel la prothèse dentaire (100) colorée est balayée optiquement pour déterminer la valeur chromatique réelle de la coloration de la prothèse dentaire (100) avec le liquide de coloration (103).

4. Procédé selon la revendication 3, où la valeur chromatique réelle déterminée est convertie en la valeur chromatique naturelle associée de la dent.

5. Procédé selon l'une des revendications précédentes, où la prothèse dentaire (100) est frittée.

6. Procédé selon l'une des revendications précédentes, où la valeur chromatique du liquide de coloration (103) est intégrée dans un ensemble de données décrivant la forme tridimensionnelle de la pièce de prothèse dentaire (100).

7. Procédé selon la revendication 6, où l'ensemble de données est obtenu sur la base d'une modélisation tridimensionnelle de la prothèse dentaire (100).

8. Procédé selon la revendication 7, où l'ensemble de données est envoyé à une station d'impression pour colorer la prothèse dentaire (100).

9. Procédé selon l'une des revendications précédentes, où la valeur chromatique du liquide de coloration (103) est superposée visuellement sur des lunettes de données à la zone prédéterminée de la pièce prothétique dentaire (100).

10. Procédé selon l'une des revendications précédentes, où la valeur chromatique du liquide colorant (103) est superposée visuellement à la zone prédéfinie de la prothèse dentaire (100) sur un appareil d'affichage portable.

11. Procédé selon l'une des revendications précédentes, où la valeur de la couleur naturelle de la dent est obtenue par un balayage coloré d'une dent et/ou d'un moignon de dent existant.

12. Procédé selon l'une des revendications précédentes, où une profondeur de couleur est associée à la zone prédéterminée (101) de la prothèse dentaire (100) affichée et la profondeur de couleur est convertie en un nombre d'applications du liquide de coloration (103).
